# EUROPEAN PATENT APPLICATION

(11) **EP 1 566 144 A1**
(43) Date of publication of application: **24.08.2005**
(21) Application number: 04003858.0
(22) Date of filing: 20.02.2004
(51) Int. Cl.: A61B 5/055, A61B 5/16

(54) **Translational sensory stimulation procedure for the stimulation of the amygdala-hippocampal complex**

(71) Applicant: Universität Basel, 4003 Basel (CH); Novartis Forschungsstiftung, Zweigniederlassung Friedrich Miescher Institute for Biomedical Research, 4058 Basel (CH)
(72) Inventor: Seifritz, Erich, 3065 Bolligen (CH); Scheffler, Klaus, 4058 Basel (CH); Lüthi, Andreas, 4054 Basel (CH)
(74) Representative: Jelsch, Emmanuel Edwin

(57) **Abstract**

The present invention relates to a novel method and to a novel system for the stimulation of the amygdala-hippocampal complex of a subject by a translational sensory stimulation procedure based on random sensory pulses. Furthermore, it relates to a novel method for the representation of the neural activity of the amygdala-hippocampal complex of a subject.

## Description

The present invention relates to a novel method and to a novel system for the stimulation of the amygdala-hippocampal complex of a subject by a translational sensory stimulation procedure based on random sensory pulses. Furthermore, it relates to a novel method for the representation of the neural activity of the amygdala-hippocampal complex of a subject.

The amygdala-hippocampal complex is a key structure in the mammalian brain that mediates emotional and related processes. The amygdala-hippocampal complex is increasingly considered as a brain structure whose physiological functions are disturbed in affective (depression) and anxiety disorders as well as related disorders such as schizophrenia, personality disorder, addiction and other mental health conditions. It plays a central role in emotional memory formation, fear processing, emotional recognition, social functions and associative learning mechanisms. Understanding the function of the amygdala-hippocampal complex is one of the major targets in cognitive neuroscience in humans and animals, and represents an important target of functional genomics as well as drug development for psychiatric conditions.

While current studies concentrate either on mechanisms in the human or in the animal brain, true translational approaches, which relate mechanisms in humans with mechanisms in animals and vice versa remained scarce. However, it is considered that translational research or translational neuroscience is an important route to understanding and treating human disease conditions (Society for Neuroscience: Translational Neuroscience Accomplishments. Society for Neuroscience (http://web.sfn.org/contentlPublications/TranslationalNeuroscience/translational.pdf), 2003)). Translational research, however, represents probably one of the most promising approaches to a better understanding of mental health conditions. While in humans, brain functions and dysfunctions can be studied in the target species, the possibilities to challenge and study such processes are limited. Translational studies allow going a step ahead using animal models, including electrophysiological, pharmacological and molecular approaches. A major problem in translational studies, however, is that functional probes usually vary significantly between human and animal experiments, which reduces the translational comparability and limits the inferences that can be carried from humans to animals and from animals to humans. For example, neural mechanisms in the amygdala of animals are classically studied using fear-conditioning experiments, in which a fear-inducing stimulus (e.g. an electrical foot shock), the unconditioned stimulus, is combined with a neutral stimulus (e.g. a soft tone), the conditioned stimulus. Such and other stimulation paradigm as are reviewed by LeDoux (Annu Rev Neurosci 2000;23:155-84).Obviously, this approach is quite limited in human research, especially in humans with affective or anxiety problems. On the other hand, amygdala and amygdala-hippocampal complex activation studies in humans are typically based on stimuli accessing higher cognitive or social functions including emotionally valenced pictures (e.g. angry faces) and other suitable stimulation paradigms as described by Zald (Brain Res Rev 2003;41:88-123).

Although the common denominator of classical probes used in animals and humans is neural activity within the amygdala-hippocampal complex, the mechanisms that lead to this effect may be quite different and thus are comparable in a translational view only with significant limitations.

In addition, previously used stimulation approaches to activate the amygdala-hippocampal complex are based on stimuli that have an intrinsic emotional value to the subject exposed to the stimulus like e.g. emotionally valence pictures, aversive sounds, pain, infant cries or laughs and the like. Furthermore, typical current approaches for stimulating the amygdala-hippocampal complex in humans and animals have the disadvantage that the subject gets habituated to the stimulus after a short period of time like approximately 2-10 seconds as described by Breiter et al. (Neuron 1996;17:875-87) and Bordi and LeDoux (J Neurosci 1992;12:2493-503). Thus, known stimuli are losing their amygdala-hippocampal complex - stimulating properties across repetitive applications over time leading to transient but not persistent neural activity alteration. A specific disadvantage of this transient neural response of the amygdala-hippocampal complex is that experimental studies during altered neural activity state of the amygdala-hippocampal complex are limited.

The object of the present invention is to provide an improved approach for the stimulation of the amygdala-hippocampal complex, which is applicable for different subjects and which does not have the above-mentioned disadvantages.

This object has been achieved with a novel method according to claim 1 and the novel system according to claim 9.

According to the invention the amygdala-hippocampal complex of a subject is stimulated by a method comprising generating and transmitting a translational sensory stimulation procedure based on random sensory pulses to said subject, which alters the neural activity of said amygdala-hippocampal complex of said subject.

### Brief description of the figures

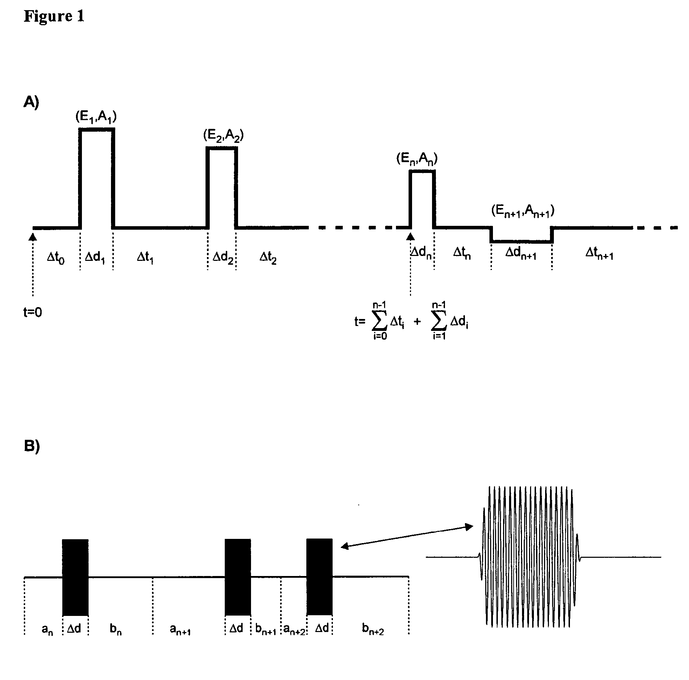

**Figure 1A)** shows a general random sensory pulse sequence. Eᵢ,Aᵢ: i-th pulse with intensity, amplitude or other feature Aᵢ; Δtᵢ: time delay between pulse Eᵢ and Eᵢ₊₁; Δdᵢ: duration of pulse Eᵢ; Aᵢ are realizations of random numbers drawn from an arbitrary probability density function p(x) defined on any interval I with x ∈ I. p(x) may be the density function of a Gaussian, Poisson, uniform or other suitable distribution. Δtᵢ and Δdᵢ are realizations of random numbers drawn from an arbitrary probability density function p(x) defined on any interval I with x ∈ I and x ≥ 0. p(x) may be the density function of a Gaussian, Poisson, uniform or other suitable distribution. Random numbers following a certain probability density function p(x) can be produced numerically, see for example W.H. Press et. al. "Numerical Recipes" Cambridge University Press 1994.
**Figure 1B)** shows as an example of random sensory pulses the random presentation of short tones (1kHz frequency and 40 milliseconds (ms) duration) with an average pulse repetition rate of 5 Hz and a corresponding average repetiton time T of T = 1/5 Hz = 200 ms. aₙ, Δd, and bₙ are time intervals, and the sum T = aₙ + Δd + bₙ defines one period of 200 ms duration, or 5Hz. Δd is the duration of the sound pulse of 1kHz frequency, set to 40 ms. The sound is switched-on and -off smoothly using a cosinusodially increasing and decreasing amplitude ramp with a duration of 3 ms. aₙ is a time interval without sound of random duration. aₙ is numerically generated for each period n as a realization of a random number derived from a uniform distribution defined within an interval of [(T/2 - Δd/2)*(1 - var), (T/2 - Δd/2)*(1 + var)]. var defines the randomness (variation of occurrence) of the sound pulses, and can bet set between 0 and 1. var = 0 results in a regular order of tones while var = 1 gives a random realization of aₙ between 0 ms and 160 ms. bₙ was calculated via bₙ = 200 ms - Δd - aₙ, using T = 200 ms.

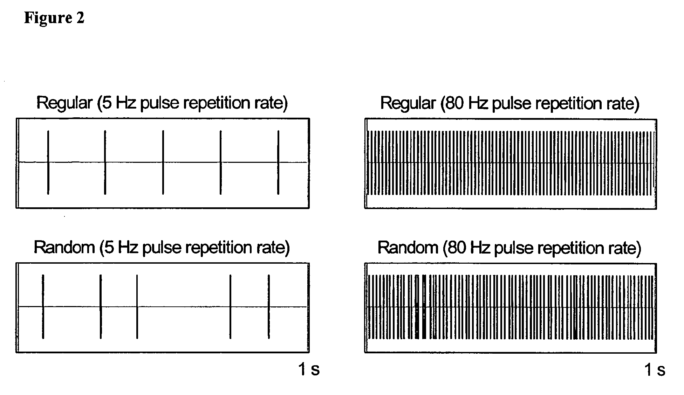

**Figure 2** shows a regular and a random sound pulse sequence with a repetition rate of 5 Hz and 80 Hz, respectively (x-axis, time; y-axis, sound level). The sound pulses with the same pulse repetition rate have the same number of pulses per time and the same sound energy.

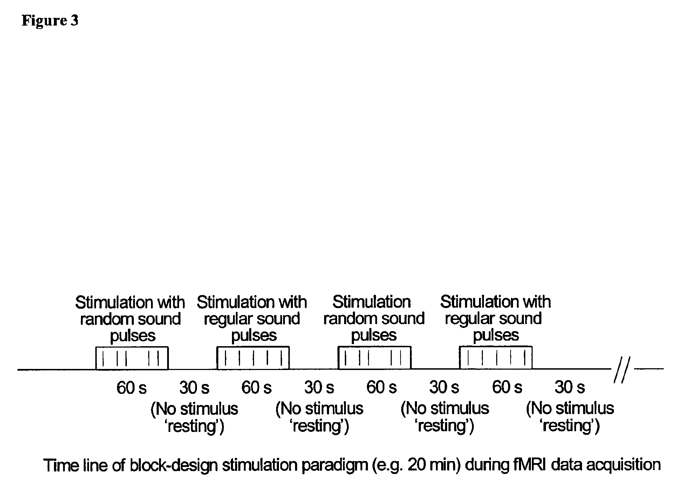

**Figure 3** shows an example of a simple block-design stimulation implemented in fMRI. The random sound pulses generated and transmitted consists of sound pulses with a repetition rate of 5 Hz, regular sound pulses were applied as well with a repetition rate of 5 Hz.

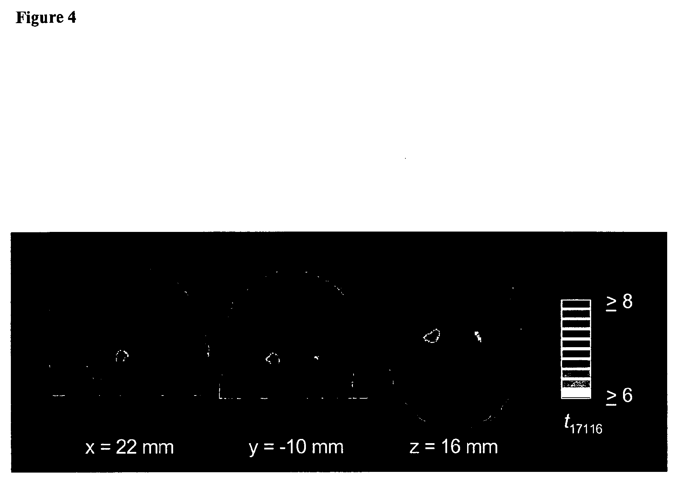

**Figure 4** shows blood oxygen level dependent (BOLD) activation by translational sensory stimulation procedure (trSP) through sound pulses in human amygdala-hippocampal complex bilaterally (right > left) in a group of 20 healthy human volunteers (map is shown in white-to-black; scaled in darkness according to gradient black-and-white coding shown on the right side of figure), as determined using functional magnetic resonance imaging (fMRI) in combination with a block-stimulation design as shown in Figure 3. Statistical evaluation based on a general linear model analysis (p-corrected < 0,001). The term "p" is the statistical term of "probability", the lower a p-value, the more statistically significant is the result of an inferential statistical test, typically, p-values below 0,05 are considered statistically significant; the term "corrected" refers to the fact that the image voxel-wise statistical tests have been corrected for the number of voxels in the entire brain slab covered during the fMRI experiment. Parametric statistical map of BOLD response is projected on sagittal (left), coronal (middle) and transversal (right) slices of a standardized brain template (Montreal Neurological Institute [MNI] standard brain; coordinates are in Talairach standard space). Slices are shown in radiological convention (right side of image shows left side of brain and vice versa). The gray-scale gradient illustrates statistical significance.

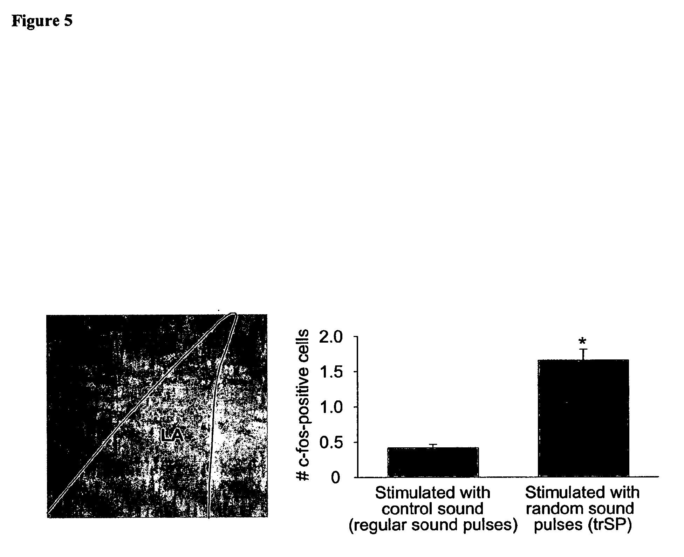

**Figure 5** shows induction of the activity-induced immediate-early gene c-Fos in the lateral amygdala of freely moving mice: Left panel, representative mouse brain slice through lateral amygdala (LA) showing the c-Fos gene product (black stains) after induction of the immediate-early gene c-Fos by trSP through sound pulses. Right panel, results of quantitative analysis of c-Fos induction by trSP (stimulated) compared to a control condition with regularly pulsed sounds in mice (n = 7/group; stimulation lasted 4 x 30 s; *, p < 0,05; 2-tailed t-test). The term "p" is as described in Figure 4; the term "t" signifies that a so-called Students-t-test has been employed for inferential statistical comparison between stimulated [exposed to random sound sequences] and control [exposed to regular sound sequences] animals. "t" is quantitative value of how strong the statistical difference between stimulated and control condition was and is used to compute the p-value.

### Translational sensory stimulation procedure (trSP)

As used herein, the term "translational sensory stimulation procedure" ("trSP") refers to a sensory stimulation procedure based on random sensory pulses applicable to different kinds of subjects, which can be applied using the same stimulus in the same way e.g. by the same experimental settings equally to different kinds of subjects.

As used herein, the term "sensory pulses" refers to any kind of pulses e.g. light pulses, tactile pulses, olfactory pulses, gustatory pulses, electric pulses or sound pulses which, if applied to a subject, will cause a physiological stimulus in that subject.

As used herein, the term "alteration of neural activity" or "neural activity altered" refers to chemical or electrochemical activity altered by a physiological stimulus caused in that subject by the trSP or directly altered by the trSP.

The alteration of neural activity of the amygdala-hippocampal complex of a subject by the trSP according to the stimulation method of the present invention can be observed by following the development of e.g. behaviour, anxiety, startle response or emotional memory formation or other amygdala-hippocampal complex-dependent physiological functions of the subject during the trSP is generated and transmitted to the subject or can be detected by non-invasive means or invasive means. Preferably, the alteration of neural activity is detected by non-invasive means or invasive means.

The random sensory pulses generated and transmitted to the subject can be e.g. light pulses, tactile pulses, olfactory pulses, gustatory pulses, electric pulses or sound pulses, preferably random sound pulses are applied. The random sensory pulses are usually generated and transmitted to the subject in a way that they are perceptible by the subject under the conditions exposed to the trSP but do not harm the subject, e.g. in case sound pulses are applied to e.g. a human subject the range of sound level is usually set between 70 and 100 db (decibel), preferably between 80 and 90 db.

The trSP based on random sensory pulses of the present invention usually consists of time-series of random sensory pulses separated by intervals during which no pulses and/or regular sensory pulses are applied like described in Figure 3 or of a time-serie of random sensory pulses, which is generated and transmitted to the subject without intervals of no pulses and/or regular sensory pulses. Whether time-series of random sensory pulses separated by intervals during which no pulses and/or regular sensory pulses are applied or a time-serie of random sensory pulses without intervals of no pulses and/or regular sensory pulses are applied depend on the way the alteration of neural activity of the amygdala-hippocampal complex of the subject is observed or detected. In case the development of e.g. behaviour, anxiety, startle response or emotional memory formation or other amygdala-hippocampal complex-dependent physiological functions of the subject is observed during the trSP generated and transmitted usually a time-serie of random sensory pulses without intervals of no pulses and/or regular sensory pulses is applied. In case the alteration of neural activity is detected by non-invasive means or invasive means normally both kind of pulses can be used except for means, which need control or reference pulses to obtain reliable data like fMRI, and other functional brain imaging methods like positron emission tomography (PET), single photon emission tomography (SPECT) as well as electrophysiological brain mapping methods such as electroencephalography (EEG), magnetoencephalography (MEG) and related methods. If such means are used, time-series of random sensory pulses separated by intervals during which no pulses and/or regular sensory pulses are usually applied.

Figure 2 shows exemplarily for sound pulses two examples of regular and random pulses with the same pulse repetition rate, the same number of pulses per time and the same pulse energy. The random sensory pulses usually have the same physical properties like the same occurrence of single pulses within a given time e.g. averaged across one or two seconds and the same pulse energy e.g. sound energy in case the sensory pulse applied is a sound pulse, as regular sensory pulses.

Regular sensory pulses consist of a periodic, predictable order of pulses. In contrast, the random sensory pulses of the trSP of the present invention consist of an order of sensory pulses which is not predictable, i.e. the occurrence of pulses is aleatory and can not be forecasted by the subject exposed to those sensory pulses based on passed order of pulses onto future pulses (the past pulses do not provide sufficient information to predict future pulses). Usually, the random sensory pulses consist of an order of pulses generated by separating single sensory pulses by a random time interval Δt, whereby after each sensory pulse, Δt is derived as a realization of a random value according to an arbitrary probability density function p(Δt) defined on an interval Δt > 0. Figure 1B shows a possible realization of a random sensory pulse sequence where the random time interval Δt between pulses is devided into two time periods bₙ and aₙ₊₁ with Δt = bₙ + aₙ₊₁. Preferably, aₙ is generated numerically for each period as a realization of a random number derived from a uniform distribution defined within an interval of [(T/2 - Δd/2) * (1 - var), (T/2 - Δd/2) * (1 + var)], where T is the mean repetition time of pulses (T = aₙ + Δd + bₙ) and var a constant between 0 and 1. bₙ is derived via bₙ = T- aₙ - Δd. var is a real number that defines the randomness (variation of occurrence) of the sensory pulses, and can be set between 0 and 1, var ∈ [0,1]. var = 0 results in a regular order of sensory pulses, while var = 1 gives a maximum random order of sensory pulses. The degree of randomness can be varied by varying var within the formula between 0 and 1. Preferably, the degree of randomness is varied between var = 0 and var = 0,9. The range for the random time interval Δt as defined in Figure 1A is usually 0 < Δt ≤1 second, preferably 0,001 < Δt ≤ 0,9 seconds, 0,01 ≤ Δt ≤ 0,5 seconds, in particular 0,1 ≤ Δt ≤0,3 seconds. The random time interval Δt does preferably not consist of a sequence of different multiples of Δt, in order to prevent any predictability of the sensory pulse time-series.

The average pulse repetition rate (as given by 1/(Δt + Δd) in Figure 1A or by 1/T = 1/(aₙ + Δd + bₙ) in Figure 1B, (x) denotes the expectation value of a random variable x) can be varied, and is usually between 1 and 100 Hz, preferably between 1 and 10 Hz, more preferably between 2 and 6 Hz. However, with faster pulse repetition rate, the duration of individual pulses must be shorter accordingly.

The duration Δd of a single sensory pulse can also be a random time interval, whereby Δd is derived after each time interval Δt as a realization of a random value according to an arbitrary probability density function p(Δd) defined on an interval Δd > 0. Δd is usually between 0,001 and 0,5 seconds, preferably between 0,02 and 0,1 seconds.

The intensity I of each single pulse can also be random whereby I is derived after each time interval Δt as a realization of a random value according to an arbitrary probability density function p(I) defined on any interval. As probability density functions p(Δt), p(Δd) and p(I) the density function of a Gaussian, Poisson, uniform or other suitable distribution can be used.

Examples for time-series of random intervals of Δt, Δd and I are given in Figures 1 A) and 1B).

Preferably, the trSp based on random sensory pulses of the present invention consists of time-series of random sensory pulses separated by intervals during which no pulses and/or regular sensory pulses are applied. In case time-series of random sensory pulses separated by intervals during which no pulses and/or regular sensory pulses are applied, they are preferably applied in a so-called block-design fashion. In a block-design fashion the time-series of random sensory pulses are typically separated by intervals during which no pulses are applied and which are followed by intervals during which regular sensory pulses are applied as shown e.g. in Figure 3. In a block design, a time period of random sensory pulses usually last between 10 seconds and 1 hour, preferably between 20 seconds and 10 minutes, more preferably between 40 seconds and 2 minutes. Usually, the time period applied for random sensory pulses and for intervals with no pulses and with regular sensory pulses are balanced, i. e. intervals during which no pulses and/or regular sensory pulses are applied usually last the same time period than the time period for random sensory pulses. The use of a block design allows to directly compare neural activity produced by two different types of stimuli (random and regular sensory pulses) by detecting the general linear contrast between different regressors (or predictors), which is particular useful if fMRI or other means which needs control or reference pulses to obtain reliable data are used as means for detecting the altered neural activity. The statistical evaluation of an effect associated with a given stimulus type is typically based on the so-called General Linear Model (GLM), which is basically a multiple regression analysis and which allows to estimate statistically the contribution of a given stimulus (in GLM used as regressor or predictor) as described in Friston et al. (Hum Brain Mapp 1995; 2:189-210) to the variance in the biological signal, which might be in case of fMRI the BOLD signal and in other means of detection other specific signals.

The subject is exposed to the translational sensory stimulation procedure of the present invention usually between 1 minute and 24 hours, preferably between 10 minutes and 2 hours more preferably between 20 minutes and 1 hour.

### Means for generating and transmitting the translational sensory stimulation procedure

Means for generating and transmitting the translational sensory stimulation procedure depend on the kind of sensory pulses applied and consist usually of a sensory output device such as a source to produce the respective pulse time-series like a compact disk player or a tape connected to a sound amplifier, in case the sensory pulses applied are sound pulses, whereby the source is controlled by e.g. a personal computer, or can be controlled by the control unit of the means for detecting the neural activity altered. Means for generating and transmitting sensory pulses to a subject, which can be used to generate and transmit the translational sensory stimulation procedure of the present invention, are known to the persons skilled in the art. The translational sensory stimulation procedure can be transmitted directly from the source to the subject or can be transmitted by an appropriate auxiliary device. In case of light pulses, transmission of light occurs usually directly to the light sensitive parts of the subject e.g. the eyes of a human or an animal are directly exposed to the light source. In case of tactile pulses, electric pulses or sound pulses usually an appropriate auxiliary device such as devices, which are in contact with the surface of the subject, electrodes or headphones are applied.

### Amygdala-hippocampal complex

The "amygdala-hippocampal complex" referred to in the present invention encompasses the amygdala plus the anterior hippocampus. The amygdala plus the anterior hippocampus consists of a group of neural nuclei comprising the so-called amygdaloid complex and the anterior hippocampus. The anterior hippocampus comprises the pes hippocampus and the anterior portion of the subiculum as defined in Bohbot et al. (Neuropsychologia 1998; 36:1217-1238). The amygdaloid complex comprises the lateral, basal, accessory basal, and centromedial nuclei as described in Sah et al. (Ann NY Acad Sci 2003; 985:67-77). The amygdaloid complex is preferably stimulated by the stimulation procedure of the present invention, whereby the lateral part is particularly stimulated. In case the subject exposed is a human, the term "amygdala-hippocampal complex" referred to in the present invention encompasses said amygdala plus the anterior hippocampus. In case the subject exposed is an animal, the term amygdala-hippocampal complex usually encompasses the amygdala and the anterior portion of the hippocampus, which are tightly interconnected as described in e.g. Pare et al. (J Neurosci 1996; 16:3334-50). The amygdala-hippocampal complex as a whole as well as part or parts of the complex can be stimulated both in in vivo and in vitro e.g. in acute brain slice preparations or in a cell culture of amygdala cells or in in vitro preparations of amygdala-hippocampal complex cells or cellular networks. Normally in in vitro preparations the stimuli used are electric pulses suitable to stimulate in vitro cell preparations.

### Subject

Subjects, which can be exposed to the stimulation procedure of the present invention, are living beings, which have developed an amygdala-hippocampal complex e.g. humans and animals, usually, humans and animals are exposed. The stimulation procedure can be applied to healthy humans, humans with a specific genetic background, which is known to be a risk factor for mental health conditions or patients with mental health problems encompassing affective and/or emotional and/or cognitive dysregulation such as e.g. patients with mood disorders, anxiety disorders, schizophrenia, personality disorders, addiction and related disorders. Animals usually exposed to the stimulation procedure are mammals like monkeys or rodents like mices, rats or rabbits and are usually laboratory animals. The animals might be healthy or might have developed behaviours, which can be used as model of mental health conditions naturally or by laboratory treatment. One might use animals showing similarities to affective/emotional dysregulation state conditions e.g. different kind of strains with altered fear-related behaviour or altered social behaviour, or animals with specific genetic background e.g. knock-out or transgenic animals, or animals treated behaviourally to show affective-like or emotionally dysregulated behaviour patterns (e.g. by early separation from mother, by induction of learned helplessness), or animals treated pharmacologically to exhibit altered behaviour and/or physiology modelling mental health problems in humans or animals with brain lesions.

The stimulation procedure of the present invention can be applied using the same stimulus in the same way e.g. by the same experimental settings equally to all subjects independent of the kind of subject exposed. Therefore, the responsiveness of the application of the stimulation procedure in different subjects can be directly compared in a truly translational approach.

### Neural activity

The alteration of the neural activity in the amygdala-hippocampal complex by the trSP can be an increase or a decrease of the neural activity, or the neural activity can be one or more times increased and thereafter be decreased or vice versa over the time-serie the subject is exposed to the stimulation procedure of the present invention. In case time-series of random and regular sensory pulses are applied the alteration caused by the random sensory pulses can be a disproportional increase (as compared to the regular stimulation procedure). Usually, the neural activity in the amygdala-hippocampal complex stimulated by the trSP is increased.

In case the neural activity altered in amygdala-hippocampal complex by the translational sensory stimulation procedure is detected by non-invasive means or invasive means, preferably, non-invasive means are used. Non-invasive means, which can be applied in the present invention are known to the person skilled in the art and are usually means for functional brain mapping selected from the group comprising the functional magnetic resonance imaging (fMRI) and related magnetic resonance imaging-based techniques, electroencephalography (EEG), magnethoencephalography (MEG), positron emission tomography (PET), infrared imaging (IR) or single photon emission computer tomography (SPECT). Related magnetic resonance imaging-based techniques which can be used are as decribed by Xiong et al. (Hum Brain Mapp 2003; 20:41-9) and Buxton (Introduction to Functional Magnetic Resonance Imaging: Principles and Techniques. Cambridge, New York, Cambridge University Press, 2002). Preferably fMRI and related magnetic resonance imaging-based techniques are used, more preferably fMRI is used. Detection of the neural activity in the brain of a subject stimulated by the translational sensory stimulation procedure of the present invention by fMRI shows that the trSP alters the neural activity specifically in the amygdala-hippocampal complex of the subject as shown in Figure 4.

The respective non-invasive means used in carrying out the present invention are known to the skilled person and are commercially available. In case fMRI is used, the neural activity within the amygdala-hippocampal complex of the subject exposed to the translational sensory stimulation procedure is usually detected by measuring e.g. blood oxygen level-dependent (BOLD) signals, regional cerebral blood flow (rCBF), regional cerebral blood volume (rCBV), regional magnetic fields, or regional electric signals. Preferably, blood oxygen level-dependent (BOLD) signals are measured, however, future developments in MRI-related technologies will enable detecting other signal sources such as regional magnetic fields, and regional electric or magnetic signals, as described e.g. in Xiong et al. (Hum Brain Mapp 2003; 20:41-9).

In case non-invasive means are applied, the means for generating and/or transmitting the translational sensory stimulation procedure are usually connected and controlled by a control unit of the respective non-invasive means used e.g. MRI-compatible audio, light, tactile, gustatory, olfactory, sound or electric pulse transmission systems. The obtained signal, however, does not have to be chronologically correlated with the stimulation procedure generated and transmitted in order to detect altered neural activity in the amygdala-hippocampal complex.

In case invasive means are used the subject is usually equipped with intracerebral electrodes in specific part of the amygdala-hippocampal complex e.g. intracerebral electrodes allowing for single cell recording, or with suitable intracerebral imaging probes like infrared probes or calcium imaging probes or the subject is sacrified within a short period of time after the subject was exposed to the translational sensory stimulation procedure and the amygdala-hippocampal complex or a part of the amygdala-hippocampal complex or cells thereof are analyzed for altered neural activity. The neural activity altered can be detected by e.g. measuring the induction of genes specific for the stimulation of the amygdala-hippocampal complex like the immediate early gene c-Fos or other suitable gene products such as zif268, CREB or Erk-1/2 within the amygdala-hippocampal complex by using usual molecular biological techniques. Preferably, the induction of genes specific for the stimulation of the amygdala-hippocampal complex, particularly the induction of the c-Fos gene product is measured.

The neural activity altered in the amygdala by the translational sensory stimulation procedure of the present invention is sustained normally for a period of at least 15 seconds, preferably at least 60 seconds, more preferably at least 5 minutes, in particular at least 20 minutes up to the whole time the subject is exposed to the translational sensory stimulation procedure of the present invention. The sustained period might be dependent of the kind of subject exposed to the translational random stimulation procedure. The neural activity altered by the translational sensory stimulation procedure of the present invention is non-habituating. i.e. every exposition of multiple expositions of the subject to the random sensory pulses alters de novo neural activity in the amygdala-hippocampal complex of the subject exposed.

The system for the stimulation of the amygdala-hippocampal complex of a subject comprising i) means for generating and transmitting a translational sensory stimulation procedure based on random sensory pulses to said subject, which alters the neural activity of said amygdala-hippocampal complex, and ii) non-invasive or invasive means for detecting the neural activity altered in said amygdala-hippocampal complex by said translational random stimulation procedure consists of the means for generating and transmitting a translational random procedure and the means for detecting the neural activity altered in said amygdala-hippocampal complex by said translational sensory stimulation procedure as described above. The translational sensory stimulation procedure is generated and transmitted to a subject as described above. The subject exposed is the same kind of subjects as described above.

A further object of the present invention is to provide a method for the representation of the neural activity of the amygdala-hippocampal complex of a subject by means for functional brain mapping, which can be applied translationally to different kind of subjects thereby allowing for a direct and simple comparison of the representation received for different subj ects.

The object has been achieved with a novel method according to claim 13 and the novel device according to claim 16.

The neural activity of the amygdala-hippocampal complex of a subject is represented by means for functional brain mapping by the following method comprising:
i) generating and transmitting a translational sensory stimulation procedure based on random sensory pulses to said subject, which alters the neural activity of said amygdala-hippocampal complex,
ii) detecting the neural activity altered in said amygdala-hippocampal complex during the transmission of said translational sensory stimulation procedure by emitting a corresponding pulse sequence for the excitation in, and read out of corresponding signals from, said subject and converting said signals into image data.

Means for functional brain mapping are usually selected from the group comprising the functional magnetic resonance imaging (fMRI) and related magnetic resonance imaging-based techniques, electroencephalography (EEG), magnethoencephalography (MEG), positron emission tomography (PET), infrared imaging (IR), single photon emission computer tomography (SPECT).

As well encompassed by the present invention is a device for the representation of the neural activity of the amygdala-hippocampal complex of a subject comprising:
i) means for generating and transmitting a translational sensory stimulation procedure based on random sensory pulses to said subject which alters the neural activity of said amygdala-hippocampal complex,
ii) means for detecting the neural activity altered in said amygdala-hippocampal complex during the transmission of said translational sensory stimulation procedure by emitting a corresponding pulse sequence for the excitation in, and read out of corresponding signals from, said subj ect and converting said signals into image data.

For the detection of the neural activity of the amygdala-hippocampal complex of the subject during the transmission of said translational sensory stimulation procedure, the subject is positioned in a usual device for functional brain mapping selected from the group mentioned above with standard equipment known to the person skilled in the art. Such devices and the way how to use them is described in Toga A. and Mazziotta J. [editors], Brain Mapping: The Methods, Elsevier-Academic Press, Amsterdam NL, 2002, and Moonen & Bandettini [editors]: Functional MRI. Berlin, Heidelberg, Springer-Verlag, 2000. Preferably, fMRI is used as the functional brain mapping method for the representation of the neural activity of the amygdala-hippocampal complex.

Further encompassed by the present invention is the use of the method according to any of claims 1 to 8 in the translational study of affective or emotional dysregulation of a subject. Affective or emotional dysregulations, which can usually be studied with the method according to any of claims 1 to 8 are e.g. affective (depression) and anxiety-related disorders as well as related disorders such as schizophrenia, personality disorder, addiction and other mental health conditions as well as dysregulations of emotional memory formation, fear processing, emotional recognition, social functions, associative learning mechanisms, and other physiological functions related to emotional regulation and dysregulation. Consequently, the present invention can be used in characterizing clinically and preclinically the above mentioned conditions both in terms of cross-sectional and longitudinal characterization, e.g. in terms of studying neural underpinnings of such diseases ("pathophysiological characterization", such as described e.g. by Phillips et al. (Biol Psychiatry 2003; 54:515-28)), of studying neural underpinnings of pharmacological and other treatment effects ("therapy monitoring", such as described e.g. by Davidson et al. (Am J Psychiatry 2003; 160:64-75)), or of studying neural underpinnings of risk factors for such diseases ("endophenotyping", such as described e.g. by Hariri and Weinberger (Br Med Bull 2003;65:259-70)).

### Examples

### Example 1

### Persistent alteration of neural activity in the amygdala-hippocampal complex of healthy humans

20 healthy volunteers were studied in the fMRI using auditory stimulation. Auditory stimuli consisted of 60-s blocks with random and regular sound pulse sequences (as shown in Figure 3). Sounds were pure tones with a carrier frequency 1 kHz and an amplitude envelope (100% modulation) resulting in a 5 Hz-repetition rate of sound pulses with a duration of 20 ms (random with var = 0,6 in the random-stimulus-condition, regular in the regular-stimulus-condition) with an average duty cycle of 50%. Sound pressure levels were set to 90 dB in all conditions. The sound energy delivered to the subjects was the same for random and regular sound pulse sequences. Auditory stimuli were presented to the subj ects bilaterally using magnetic resonance imaging-compatible sound delivery system (Commander XG, www.mrivideo.com). In order to map out the time-course of activation across the 60-s duration of the stimulus epoch, image volumes were acquired with brief inter-volume intervals of 2.14 s. Image acquisition produced a banking background noise of approximately 100 dB, however, the noise reduction by the headphones of approximately 30 dB and the spectral composition of the scanner noise enabled a clear perception of experimental stimuli.

Random and regular stimulus sequences were implemented in a block-design alternating between stimulation (60 s) and resting (30 s) conditions as shown in Figure 3. Subjects were instructed to passively listen to the sound stimuli, however they were not asked to carry out any output tasks or to make judgments about the stimuli ('passive listening' without explicit rating or any task other than listening, such as e.g. in Critchley et al, (Hum Brain Mapp 2000; 9:93-105). Magnetic resonance images were acquired on a 1.5 T Symphony scanner equipped with a circularly polarized head coil. Anatomical *T*_{*1*}-weighted volumes were obtained with a three-dimensional magnetization prepared rapid acquisition gradient echo sequence at a voxel size of 1 mm³ (repetition time *TR*, 9.7 ms; echo-time *TE,* 4 ms). Functional *T*_{*2*}*-weighted images were acquired using gradient-recalled echo-planar imaging (*TE*, 54 ms; *TR*, 2675 ms; inter-slice time, 107 ms), which is a standard fMRI sequence to detect changes in regional blood oxygenation level-dependent (BOLD) signal which is accepted to index neural activity (see e.g. Logothetis et al., Nature 2001; 412:150-7). A series of 865 functional near-whole-brain volumes consisting of 20 contiguous oblique slices 4 mm thick (field of view, 180 x 180 mm²; matrix, 64 x 64 pixels) were acquired. The first 8 volumes were discarded to obtain steady state regarding longitudinal magnetization and scanner-induced auditory excitation.

Images were postprocessed using Brain Voyager (www.braininnovation.com). The functional time-series were corrected for slice-acquisition time through sinc interpolation, realigned with their corresponding *T*_{*1*}-volumes, warped into Talairach-space, resampled into 3-mm isotropic voxels, motion-corrected using Levenberg-Marquart's least square fit for six spatial parameters, corrected voxelwise for linear drifts, and smoothed using a 6-mm full-width at half-maximum gaussian kernel. Condition-specific stimulus boxcar functions were convolved with a gamma-kernel to model the hemodynamic response behaviour. The cortical areas responding to auditory stimulation were identified by applying general linear model analyses to z-transformed time-series in each image voxel and used random and regular sound sequence stimulus conditions as explanatory variables. Stimulus-specific effects were calculated using the general linear model (GLM) contrast. These contrasts were voxel-wise Bonferroni-corrected (*p* < 0,05). Statistical maps were superimposed on anatomical sections and inflated cortical surfaces of the standardized Montreal Neurological Institute *T*_{*1*}-weighted brain template (www.bic.mni.mcgill.ca).

Random and regular sound pulses produced blood oxygen level-dependent (BOLD) signal change in auditory cortex; however, in auditory cortex the BOLD responses to the two stimulus types were not statistically significantly different. In comparing the brain activity induced by random vs. regular sound pulse stimuli, a significant general linear contrast bilaterally in the amygdala and the amygdala-hippocampal complex was found, whereby the differential contrast was stronger in the right compared to the left amygdala and amygdala-hippocampal complex (Figure 4).

### Example 2

### Induction of c-Fos in the amygdala of freely moving mice

Adult male C57 B1/6 mice (RCC) were housed individually in Plexiglas cages and were maintained on a free feeding regimen with a 12/12 h light/dark schedule. All studies took place during the light portion of the cycle.

Experimental Design: Before training, mice were habituated to being handled during 4 days (5 minutes per day), transported from the colony room to the experimental chamber and familiarized with a neutral context consisting of a square transparent plexiglas box (27 cm side X 72 cm high) with a grid floor made of stainless steel rods. The whole system was placed inside a sound attenuating and temperature-regulated Plexiglas cubicle. A speaker was positioned on top of the square transparent box. This context was cleaned with 70 % ethanol before and after each animal. On the training day, mice were divided into 3 groups. Mice of the Regular group (REG group, n = 6 mice) were submitted to 4 presentations (inter-trial interval: 30 - 240 s) of a series of 5 kHz bips (duration Δd = 20 ms) regularly distributed at 5 Hz for 30 s (constant aₙ = bₙ = 90 ms). Mice of the Random group (RAND group, n = 7 mice) were submitted to 4 presentations (inter-trial interval: 30-240 s) of a series of 5 kHz bips (duration Δd = 20 ms) randomly distributed at an average frequency of 5 Hz for 30 s (var = 0,8, aₙ randomly uniformly distributed within an interval of [(100 - 10)(1 - var),(100 - 10) * (1 + var)] ms = [18,162] ms, and bₙ = 200 ms - aₙ - Δd as shown in Figure 1B).

**Immunohistochemistry:** Mice were deeply anesthetized using isoflurane (5 % in oxygen.) 2 hrs after the last presentation of an auditory stimulus and perfused transcardially with ice-cold solutions of 9 g/l NaCl followed by 4% of paraformaldehyde in phosphate buffer (PB; pH 7.4). After post-fixation overnight in the same fixative at 4°C, coronal sections (50 µm) were cut on a vibratome (Leica) and collected in PB. Free-floating sections were rinsed in phosphate buffer saline (PBS) and pretreated with 0,3% H202 in PBS to reduce endogenous peroxidase activity. After four rinses, sections were incubated in a blocking solution (3% bovine serum albumin (BSA)/0,2% Triton X100) for 1 hour (hr) at room temperature (RT). Then, they were incubated in primary polyclonal rabbit anti-c-Fos (Oncogene Research Products; 1:20000 dilution) antibody in the blocking solution overnight at RT. Subsequently, sections were washed with PBS and incubated for 2 hrs at RT with biotinylated goat anti-rabbit IgG (Vector Laboratory; 1:400 in PBS) followed by 2 hrs at RT in the avidin-biotin peroxidase complex (Vectastain Elite kit, Vector Laboratories). Sections were rinsed in PBS and then PB. The peroxidase reaction end-product was visualized by incubating sections in 0,1 M PB containing 3,3' diaminobenzidine tetrahydrochloride (DAB, 0,037%) as chromogen and hydrogen peroxide (0,015%) for 20 min. Finally, immunolabeled sections were washed in PB, mounted on gelatin-coated slides, dehydrated and coverslipped.

**Data analysis:** The quantification of c-Fos positive cells was carried out at a X 10 magnification, which yielded a field of view of 849 X 637 µm. At least three serial sections were digitized and analyzed using a computerized image analysis system (Biocom, Visiolab 2000, V4.50). The number of nuclei was quantified in the following areas of interest [according to Franklin and Paxinos (1996); - signifies posterior to bregma] : amygdala (BL and LA, bregma -1.3 to -1.9 mm). The counting was performed in an area of the same shape and size for each brain region. Nuclei were counted individually and expressed as number of c-Fos positive nuclei per mm2. At all stages, the experimenter was blind to the experimental groupings. Statistical analysis of immunocytochemical studies were performed by unpaired two-tailed student's *t*-test at the p < 0,05 level of significance. Data are presented as group means ± SEM (standard error of mean). Figure 5 (left) shows a slice through the lateral amygdala of a mouse that has been stimulated (4 x 30 s) using trSP. Results of quantitative analysis of c-Fos induction by trSP (stimulated) compared to a control condition with regularly pulsed sounds indicate alteration of the neural activity of the lateral amygdala by the trSP.

### Example 3

### Persistent alteration of neural activity in the lateral amygdala and closely connected portions of the hippocampus of freely moving mice

Data from electrophysiological trials using extracellular single unit recordings in lateral amygdala (LA) and closely connected portions of the hippocampus of freely moving mice show that stimulation with random sound sequences (trSP), compared to corresponding regular sound sequences, increases neural activity in LA. Single unit recordings were performed according to standard procedures used in freely moving rats (as described e.g. by Quirk et al. (Neuron 1997; 19:613-24) and Neugebauer and Li. (J Neurophysiol 2003; 89:716-27)).

## Claims

1. A method for the stimulation of the amygdala-hippocampal complex of a subject comprising generating and transmitting a translational sensory stimulation procedure based on random sensory pulses to said subject, which alters the neural activity of said amygdala-hippocampal complex of said subject.

2. The method according to claim 1, further comprising detecting the neural activity altered in said amygdala-hippocampal complex by said translational sensory stimulation procedure by non-invasive means or invasive means.

3. The method according to claim 1 or 2, wherein the random sensory pulses are random sound pulses.

4. The method according to any of claim 1 to 3, wherein the random sensory pulses consist of an order of pulses generated by separating single sensory pulses by a random time interval Δt, whereby after each sensory pulse, Δt is derived as a realization of a random value according to an arbitrary probability density function p(Δt) defined on an interval Δt > 0.

5. The method according to any of claim 1 to 4, wherein said translational sensory stimulation procedure based on random sensory pulses consists of time-series of random sensory pulses separated by intervals during which no pulses and/or regular sensory pulses are applied.

6. The method according to claim 5, wherein said time-series of random sensory pulses separated by intervals during which no pulses and/or regular sensory pulses are applied in a block-design fashion.

7. The method according to any of claim 2 to 6, wherein the non-invasive means are means for functional brain mapping selected from the group comprising the functional magnetic resonance imaging (fMRI) and related magnetic resonance imaging-based techniques, electroencephalography (EEG), magnethoencephalography (MEG), positron emission tomography (PET), infrared imaging (IR), or single photon emission computer tomography (SPECT).

8. The method according to any of claim 1 to 7, wherein the altered neural activity in said amygdala-hippocampal complex is sustained for a period of at least 15 seconds.

9. A system for the stimulation of the amygdala-hippocampal complex of a subject comprising:
i) means for generating and transmitting a translational sensory stimulation procedure based on random sensory pulses to said subject which alters the neural activity of said amygdala-hippocampal complex,
ii) non-invasive or invasive means for detecting the neural activity altered in said amygdala-hippocampal complex by said translational random stimulation procedure.

10. The system according to claim 9, wherein the random sensory pulses are random sound pulses.

11. The system according to claim 9 or 10, wherein the random sensory pulses consist of an order of pulses generated by separating single sensory pulses by a random time interval Δt, whereby after each sensory pulse, Δt is derived as a realization of a random value according to an arbitrary probability density function p(Δt) defined on an interval Δt > 0.

12. The system according to any of claim 9 to 11, wherein the non-invasive means are selected from the group comprising the functional magnetic resonance imaging (fMRI) and related magnetic resonance imaging-based techniques, electroencephalography (EEG), magnethoencephalography (MEG), positron emission tomography (PET), infrared imaging (IR), or single photon emission computer tomography (SPECT).

13. A method for the representation of the neural activity of the amygdala-hippocampal complex of a subject by means for functional brain mapping comprising:
i) generating and transmitting a translational sensory stimulation procedure based on random sensory pulses to said subject, which alters the neural activity of said amygdala-hippocampal complex,
ii) detecting the neural activity altered in said amygdala-hippocampal complex during the transmission of said translational sensory stimulation procedure by emitting a corresponding pulse sequence for the excitation in, and read out of corresponding signals from, said subject and converting said signals into image data.

14. The method according to claim 13, wherein the random sensory pulses consist of an order of pulses generated by separating single sensory pulses by a random time interval Δt, whereby after each sensory pulse, Δt is derived as a realization of a random value according to an arbitrary probability density function p(Δt) defined on an interval Δt > 0.

15. The method according to claim 13 or 14, wherein the neural activity is detected by detecting the blood oxygen level-dependent (BOLD) signal with functional magnetic resonance imaging (fMRI) in said subject.

16. A device for the representation of the neural activity of the amygdala-hippocampal complex of a subject comprising:
i) means for generating and transmitting a translational sensory stimulation procedure based on random sensory pulses to said subject, which alters the neural activity of said amygdala-hippocampal complex,
ii) means for detecting the neural activity altered in said amygdala-hippocampal complex during the transmission of said translational sensory stimulation procedure by emitting a corresponding pulse sequence for the excitation in, and read out of corresponding signals from, said subject and converting said signals into image data.

17. The use of the method according to any of claims 1 to 8 in the translational study of affective or emotional dysregulation of a subject.
